# EUROPEAN PATENT SPECIFICATION

(11) **EP 0 890 361 B1**
(45) Date of publication and mention of the grant of the patent: **29.12.2004**
(21) Application number: 96935432.3
(22) Date of filing: 23.10.1996
(51) Int. Cl.: A61K 38/18, A61P 35/00

(54) **HGF mutant and its use as an anticancer agent**
HGF Mutant und dessen Verwendung als Antikrebsmittel
Mutant du HGF et son utilisation comme agent anticancéreux

(30) Priority: 24.10.1995 JP 30072895
(43) Date of publication of application: 13.01.1999
(73) Proprietor: Nakamura, Toshikazu, Kyoto 606-8333 (JP)
(72) Inventor: Nakamura, Toshikazu, Kyoto 606-8333 (JP)
(74) Representative: von Kreisler, Alek, Dipl.-Chem.
(86) International application number: PCT/JP1996/003105
(87) International publication number: WO 1997/016205

(56) References cited:
- WO-A-93/23541
- WO-A-94/06909
- WO-A-96/40914
- JP-A- 6 025 010
- SCIENCE, Vol. 254, No. 5036, 1991, pages 1382-1385, XP002002187.
- J. BIOL. CHEM., Vol. 268, No. 23, 1993, pages 17145-17150, XP000942254.

## Description

### TECHNICAL FIELD

The present invention relates to an anti-cancer agent. More specifically, it relates to a novel anti-cancer agent containing α-chain of HGF (hepatocyte growth factor) as an active ingredient, capable of suppressing carcinoma, especially invasion and metastasis of carcinoma, on the basis of its c-Met/HGF receptor-antagonist activity.

### BACKGROUND ART

Control of cancer is a most important subject on today's medicine, and new cancer therapy and new anti-cancer agents are topics of the greatest interest among medical and pharmaceutical researchers. At the present, the fatal cause number 1 in Japan is the cancer, and many new patients are found every year.

As anti-cancer agents used in chemotherapy, aside from the conventional alkylating agents, metabolic antagonists, and antibiotics, microorganism-derived bioreactive modification substances represented by Picibanil (trade name of Chugai Pharmaceutical, Japan) and Krestin (trade name of Sankyo Pharmaceutical, Japan) once flourished. Existing compounds such as alkylating agents originally made use of cytotoxicity, and the uses were considerably limited owing to manifest side effects, but Picibanil and other later bioreactive modification substances have actions of enhancing the immune function of the body and expel cancer cells. However, as their limits have come to be known, the attention has been turned to bio-active proteins derived from higher animals such as interferon, interleukin 2, and TNF (tumor necrosis factor). Yet, their action spectra were actually by far broader than initially estimated, and the belief that they were free from side effects was lost.

In this background, it is at least true that the treatment of cancer is changing its direction, that is, from the conventional evaluation of mere fighting against the cancer focus to the concept considering "quality of life" to evaluate the treatment in the process of improvement of total function of the body, and HGF discovered by the present inventor has been already reported that it is an active ingredient for anti-cancer agent (Japanese Laid-open Patent No. 6-25010).

As mentioned above, the existing anti-cancer agents are far from a decisive remedy in consideration of the side effects and doubts about the anti-cancer action itself, and the bio-active proteins of the next generation are mainly factors relating to the immune system as far as developed in the past, and are not always expected to be used widely as ultimate anti-cancer agents. Accordingly, among the bio-active proteins produced in the body, in particular, among those definite in physiological action and researched sufficiently of the spectrum of activity, it is required to discover a true anti-cancer agent. Especially, since the bio-active proteins, interferons and interleukins developed so far are mostly factors relating to the immune system, biological factors having a completely different action seems to be important as a future anti-cancer agent.

In Japan, the cancer is the fatal cause number 1 as mentioned above, but it may be dared to say that its risk depends on invasion and metastasis of cancer cells. Several growth factors have been reported to be related to the ability of invasion and metastasis of cancer cells, and recently HGF has been disclosed to be a strong factor for inducing invasion and metastasis ability to various cancer cells (H. Shimura et al., J. Jap. Cancer Res. 86, 662-669, 1995). Actually, the invasion ability of lung cancer and gallbladder cancer known to be extremely high in malignancy has been confirmed to be induced depending on HGF, and HGF level in the primary tissues of lung cancer is known to be a risk factor closely correlating with the malignancy and mortality of lung cancer.

HGF is a protein which has been discovered as a factor for proliferating liver parenchymal cells in vitro (Biochem Biophys Res Commun, 122, 1450, 1984; Proc. Natl. Acad. Sci. USA, 83, 6489, 1986; FEBS Letter, 22, 311, 1987; Nature, 342, 440, 1989; Proc. Natl. Acad. Sci. USA, 87, 3200, 1990). This HGF first discovered as a factor for specifically proliferating liver parenchymal cells has been disclosed to show various activities in the body such as tissue wound healing as a result of recent studies by the present inventor and many other researchers, and it is highly expected to be applied into remedies in humans and animals, as well as the subject of study. Concerning a receptor of such HGF, the recent studies unveiled that the c-Met prot oncogene codes HGF receptor (Bottaro et al., Science, 251, 802-804, 1991; Naldini et al., Oncogene, 6, 501-504, 1991).

It is thus clarified that HGF is a factor for powerfully inducing invasion and metastasis ability to various cancer cells, and development of antagonist and inhibitor for specifically blocking the invasion and metastasis ability of cancer cells by HGF seems to be an extremely important subject of study from the viewpoint of control of cancer

### DISCLOSURE OF THE INVENTION

As a result of intensive studies from such viewpoint, the inventor discovered a substance having an activity for suppressing the invasion and metastasis ability of cancer cells induced by HGF (that is, the antagonist activity to the c-Met/HGF receptor of cell), and found that his substance suppresses the invasion and metastasis ability of cancer cells and is hence extremely useful as an anti-cancer agent, and thereby completed the present invention. It is therefore an object of the invention to present an extremely useful medicine as an anti-cancer agent on the basis of the antagonist activity of c-Met/HGF receptor of cell.

That is, the invention provides an anti-cancer agent containing a protein having the following physicochemical properties and physiological activities (hereinafter referred to as α-fragment) as an active ingredient:
a) composed of the α-chain of HGF obtainable by digesting HGF with elastase;
b) having molecular weight of 55-69 kDa; and
c) having antagonist activity of c-Met/HGF receptor.

Other embodiments of the present invention relate to the use of the α-fragment as defined above for manufacturing an anti-cancer agent.

### BRIEF DESCRIPTION OF THE DRAWINGS

Fig. 1 is a chromatogram obtained when enzyme-digested HGF is purified by reverse-phase HPLC.
Fig. 2 is a photograph of electrophoresis showing the result of electrophoresis (SDS-PAGE, in reducing condition) of enzyme-digested HGF purified by reverse-phase HPLC.
Fig. 3 is a photograph showing the scattering effect of α-fragment on MDCK cells.
Fig. 4 is a photograph showing the scattering effect of α-fragment on MDCK cells in coexistence of HGF.
Fig. 5 is a graph showing the effects of HGF, EGF and α-fragment on DNA synthesis of rat liver cells. In the graph, (a) is a system by adding HGF, (b) is a system by adding α-fragment, (c) is a system by adding α-fragment in coexistence of 5 ng/ml of HGF, and (d) is a system by adding α-fragment in coexistence of 10 ng/ml of EGF.
Fig. 6 is a photograph showing the effect of α-fragment on invasion of GB-d1 cells.
Fig. 7 is a photograph showing the effect of α-fragment on invasion of GB-d1 cells in coexistence of HGF.
Fig. 8 is a graphic expression of results of Fig. 6 and Fig. 7.

### BEST MODE OF CARRYING OUT THE INVENTION

As mentioned above, the inventor has been studying growth factors of liver parenchymal cells for years, and has succeeded in isolation and purification of HGF. Initially, HGF is a polypeptide discovered to be a factor for promoting proliferation of live parenchymal cells, and it is discovered by the inventor that it functions also as motogen for promoting cell motility, in addition to the function of cell growth control (T. Nakamura, Prog. Growth Factor Res., 3, 67-85, 1991). Promotion of cell motility by HGF is related with decrease of adhesion between cells through cadherin by phosphorylation of β-catenin, and information transmitting system through activation of rho small G protein. More recently, it has been disclosed that p125FAK (focal adhesion kinase) is positioned downstream of rho, so that transient phosphorylation of p125FAK is caused by HGF (K. Matsumoto et al., J. Biol. Chem. 269, 31807-31813, 1994). After stimulation of HGF, phosphorylation of p125FAK contributes to initial formation of focal adhesion and reconstruction of cell skeleton, and in promotion of cell motility by HGF, it is considered that the interaction of cells and matrix is regulated through the p125FAK.

It has been traditionally known that the proliferation and invasion and metastasis ability of cancer cells depend much on the interaction of cancer cells and surrounding stromal cells (tumor-host relationship). The inventor unveiled that HGF derived from the host interstitium and HGF-inducing factor derived from cancer cells (injulin) contribute to aggravation of cancer (proliferation, invasion and metastasis) (K. Matsumoto et al., Gann Monograph No. 42: Growth Factors: Cell growth, Morphogenesis and Transformation, CRC press 92-112, 1994; K. Rygaard et al., Intern. J. Oncology, 2, 991-996, 1993; W. G. Jiang et al., Clin. & Exp. Metastasis, 11, 235-242, 1993; S. P. Seslar et al., Cancer Res., 58, 1233-1238, 1993; N. Rahimi et al., DNA and Cell Biol., 13, 1189-1197, 1994; S. Bellusci et al., Oncogene, 9, 1091-1099, 1994).

Gallbladder cancer is generally a malignant cancer high in metastasis rate and mortality rate. Human gallbladder cancer cells show a high invasion in the stromal cells of the host, but will not invade into the gel in the culture on collagen gel in vitro. However, in co-cultivation between the normal stromal fibroblast and collagen gel, gallbladder cancer cells actively invade into the gel, and the invasion ability of gallbladder cells is induced by the interaction with the stromal cells through a liquid factor. Moreover, invasion of cancer cells in the co-cultivation is completely inhibited by the addition of anti-HGF antibody, and the stromal-derived invasion factor is found to be HGF. This invasion of gallbladder cancer cells into the gel is not induced by representative growth factors such as EGF, TFG-β, PDGF and bFGF, and it is specific to HGF. On the other hand, gallbladder cancer cells produce and secrete factors for inducing HGF production of stromal fibroblast, and the substance of this HGF inducer was found to be IL-1β. The substance of the stromal-derived invasion factor not only in the human gallbladder cancer cells, but also in cells of many carcinomata such as oral mucosal epithelial cancer cells is found to be HGF (K. Rygaard et al., Intern. J. Oncology, 2, 991-996, 1993; W. G. Jiang et al., Clin. & Exp. Metastasis, 11, 235-242, 1993; S. P. Seslar et al., Cancer Res., 58, 1233-1238, 1993; N. Rahimi et al., DNA and Cell Biol., 13, 1189-1197, 1994).

It is said that the mortality by cancer can be decreased by preventing invasion and metastasis of cancer cells. More than 80% of cancer is carcinoma, and most of them are HGF-target cells expressing c-Met/HGF receptor. It is hence important to develop an antagonist for blocking the invasion and metastasis function of cancer cells caused by HGF.

The inventor disclosed the following points in the sequential studies on HGF. First of all, HGF is a heterodimer comprising about 69 kDa of α-chain and about 34 kDa of β-chain. The α-chain of HGF contains a hairpin domain at N-terminal and four cringle domains, and the β-chain contains a serine protease-like domain, and hence HGF is a growth factor having a very unique domain structure. The inventor already prepared various deletion-type HGFs eliminated domain structures in the HGF molecule by gene engineering technique, and disclosed that the N-terminal hairpin and first and second cringle domains in the α-chain are a minimum domain binding to c-Met/HGF receptor. Therefore, by genetically introducing the mutation into this minimum receptor binding domain, it is considered possible to manufacture HGF receptor-antagonist. Hitherto, in studies of factors for suppressing invasion and metastasis of cancer cells, the target was mainly the matrix protease derived from cancer cells, but effective substance for blocking invasion and metastasis of cancer cells has not been unveiled yet.

By contrast, the object of the invention is to block the signal for inducing invasion and metastasis of cancer cells at the upstream side, and it is a major feature that it is based on a completely new strategy, and this is a pioneering invention.

The anti-cancer agent of the invention contains the protein (α-fragment) having the above-mentioned physicochemical properties and physiological activities as an active ingredient, and this protein can be obtained, for example, by a method of decomposing HGF enzymatically, a method of preparing by gene engineering technique and a method of preparing chemically.

As to HGF used in the enzymatic decomposition method, HGF prepared by various methods may be used. Many methods are known to prepare HGF, and, for example, HGF can be obtained by extraction and purification from organs such as liver, spleen, lung, bone marrow, brain, kidney and placenta, blood cells such as platelets and leukocytes, plasma and serum of mammals such as rat, cow, horse and sheep (FEBS Letters, 224, 312, 1987; Proc. Acad. Sci. USA, 86, 5844, 1989).

Also, it is possible to obtain HGF by cultivation of primary culture cells or cell lines producing HGF, followed by separation and purification from the culture product (e.g. culture supernatant, cultured cell). Further, HGF can be obtained by gene engineering method which comprises recombining the gene coding HGF with a proper vector, inserting it into a proper host cell to give a transformant, and separating the desired recombinant HGF from the culture supernatant of the transformant (e.g. Nature, 342, 440, 1989, Japanese Patent Kokai Nos. 111383/1993 and 255096/1991, Biochem. Biophys. Res. Commun., 163, 967, 1989).

The host cell is not specifically limited, and various host cells conventionally used in gene engineering methods can be used, which are, for example, Escherichia coli, Bacillus subtilis, yeast, filamentous fungi, and plant or animal cells.

More specifically, the method of extracting and purifying HGF from live tissues is, for example, to administer carbon tetrachloride to a rat intraperitoneally, remove a liver from the rat with hepatitis, grind it, and purify by the ordinary protein purifying technique such as gel column chromatography using S-Sepharose and heparin Sepharose, or HPLC.

Further, by the gene engineering method, animals cells such as Chinese hamster ovary (CHO) cells, mouse C127 cells, monkey COS cells, Sf (Spodoptera frugiperda) cells and the like are transformed with the gene coding the amino acid sequence of HGF, and HGF can be obtained from the culture supernatant of the transformants. Incidentally, HGF derived from either humans or mammals can be used, but HGF derived from humans are preferred, and human recombinant HGF (Japanese Patent Kokai No. 111383/1993) is more preferable.

As to HGF thus obtained, as far as substantially effective as HGF, there are possibilities that a part of the amino acid sequence will be deleted or substituted with other amino acid(s), that another amino acid sequence is partially inserted, that 1, 2 or more amino acids are attached to the C and/or N terminals, or that sugars are similarly deleted or substituted.

Enzymatic decomposition of HGF is carried out by, for example, digesting HGF by using enzyme such as elastase. Successively, the digestive product is purified by a conventional protein purification method such as high performance liquid chromatography, and a low molecular HGF composed of 55-69 kDa fragment containing α-chain is isolated, so that the protein (α-fragment) having the above-mentioned physicochemical properties and physiological activities is obtained.

α-fragment of the invention is not limited to those obtained by the above methods alone, but may be prepared chemically according to the conventional peptide synthesis method. Also, α-fragment can be obtained by the gene engineering method mentioned above by using genes coding the amino acid sequence of α-fragment.

As shown in Examples below, α-fragment is disclosed to have the antagonist activity of c-Met/HGF receptor for blocking the mitogen and motogen activity of HGF, and suppress the invasion of cancer cells. Therefore, the agent of the invention mainly composed of α-fragment is useful as the anti-cancer agent for humans and mammals (for example, cow, horse, pig, sheep, monkey, dog, cat), especially as invasion suppressant and metastasis suppressant of cancer cells.

Other embodiments of the present invention relate to the use of the α-fragment as defined herein before for manufacturing an anti-cancer agent.

The anti-cancer agent of the invention is prepared in various dosage forms (for example, liquid, solid, capsule), and it is generally prepared as an injection of the active ingredient of α-fragment only, or together with a conventional carrier, or as an oral preparation together with a conventional carrier. The injection may be prepared by a conventional method, for example, α-fragment is dissolved in a proper solvent (for example, sterilized water, buffer solution, physiological saline), filtered to be sterilized, and is contained in an aseptic container. The content of α-fragment in the injection is adjusted usually to 0.0002 to 0.2 (w/v%), or preferably 0.001 to 0.1 (w/v%). As the oral preparation, it is prepared into, for example, tablet, granule, fine granule, powder, soft or had capsule, liquid, emulsion, suspension, syrup, and such dosage forms may be prepared by the conventional manufacturing methods. The content of α-fragment in the preparation may be properly adjusted according to the dosage form and the applicable diseases.

In pharmaceutical manufacture, preferably, a stabilizing agent may be added, and examples of such stabilizing agent may include, among others, albumin, globulin, gelatin, mannitol, glucose, dextran, and ethylene glycol. The preparation of the invention may also contain other additives necessary for manufacture, such as excipient, dissolving aid, antioxidant, pain-alleviating agent and isotonic agent. In liquid preparation, it is preferred to store it under frozen conditions or after the removal of water by a process such as freeze-drying. The freeze-dried preparation is used by dissolving again by adding distilled water for injection before use.

The preparation of the invention is to be administered through a proper route according to the dosage form. For example, the injection can be administered intravenously, intraarterally, subcutaneously and intramuscularly. The dose is properly adjusted depending on the symptom, agent, or body weight of the patient, and usually 0.01 mg to 100 mg of α-fragment is used, which is administered once a day or in several divided portions.

### INDUSTRIAL APPLICABILITY

In the invention, the active ingredient of α-fragment has a specific effect to suppress invasion or metastasis of cancer cells such as gallbladder cancer, lung cancer and others, which are highly metastatic and result in a high lethality. Therefore, the anti-cancer agent of the invention may be used in treatment and prevention of cancer, and are extremely useful clinically.

### EXAMPLES

The invention is more specifically described below by referring to Examples and Test Examples.

### Example 1

### Isolation and refining of α-fragment of HGF

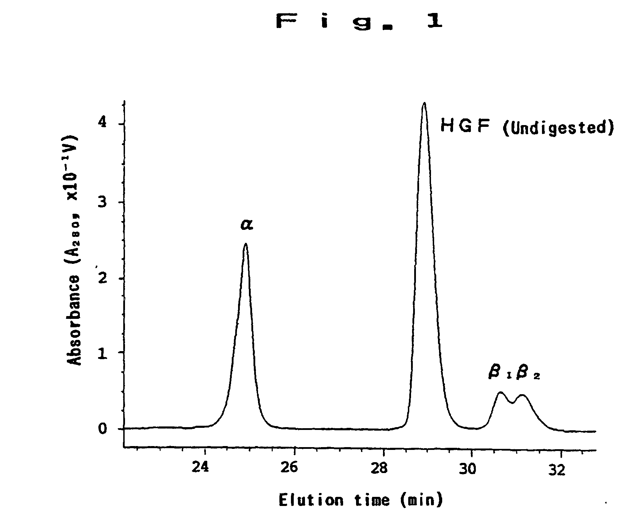

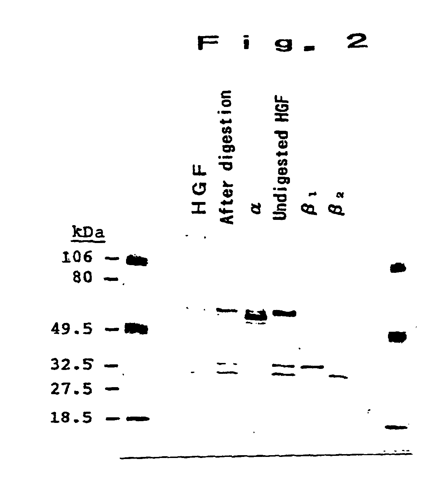

900 mg of recombinant HGF was digested in elastase for 1 hour, and it was purified in reverse-phase HPLC (C4). Its chromatogram is shown in Fig. 1. As shown in Fig. 1, four peaks were obtained, and the first peak was identified to be α-fragment, the second peak to be undigested HGF, and third and fourth peaks to be β-1 and β-2, respectively. Further by a freeze-drying system, the solvent was removed, and each of fractions was collected and suspended again in distilled water. The obtained substances were found to be α and β-fragments by SDS-PAGE (see Fig. 2). As a result of determination of protein, 178 mg of α-fragment was obtained.

### Example 2

### Analysis of action of motogen using MDCK cells

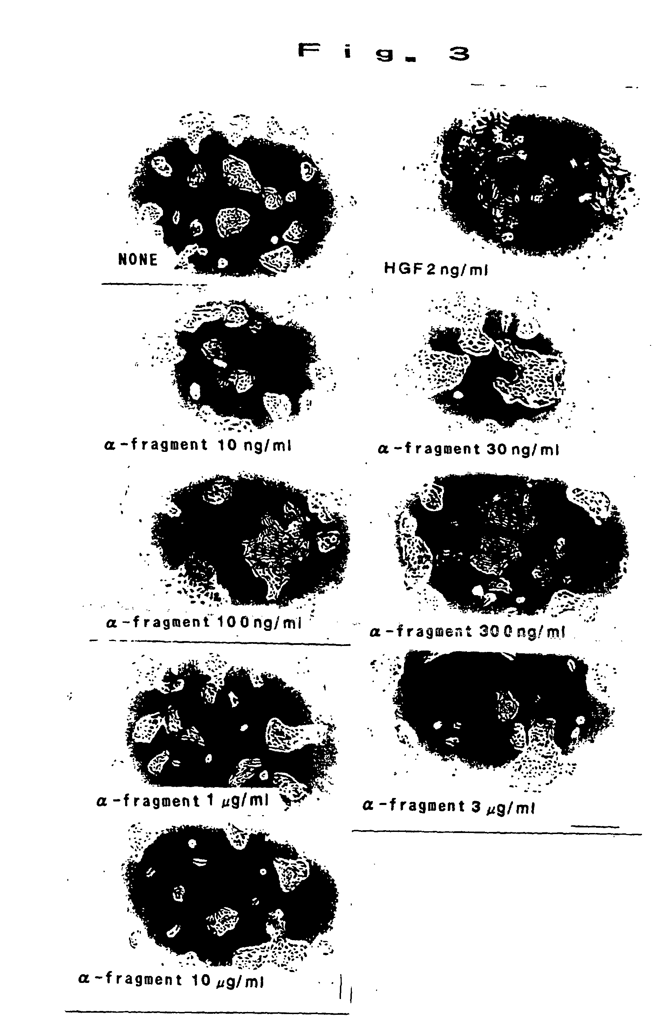

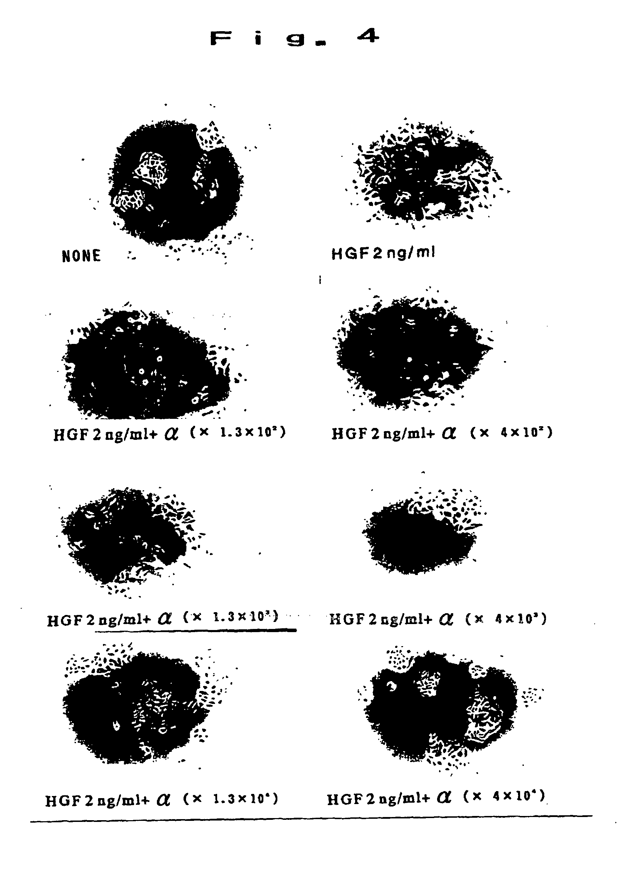

MDCK cells were prepared in 2 x 10⁴ cells/ml in 10% FBS added DMEM culture medium, and seeded in 48 well plates at 250 ml/well. By adding α-fragment alone in a range of 10 ng/ml to 10 mg/ml and cultivating for 24 hours, the surface was observed by a phase-contrast microscope. Results are shown in Fig. 3. As shown in Fig. 3, scattering action was not recognized in α-fragment. In succession, adding simultaneously with 2 ng/ml of HGF, the inhibitory effect on HGF was studied. Results are shown in Fig. 4 (in the photograph, the indication of "α" denotes the concentration of α-fragment (magnification), same hereinafter). As shown in Fig. 4, when exceeding the concentration of 1000 times, inhibition of scattering was observed dose-dependently. Thus, α-fragment was strongly suggested to be an antagonist of HGF.

### Example 3

### Analysis of action as mitogen using rat liver cells

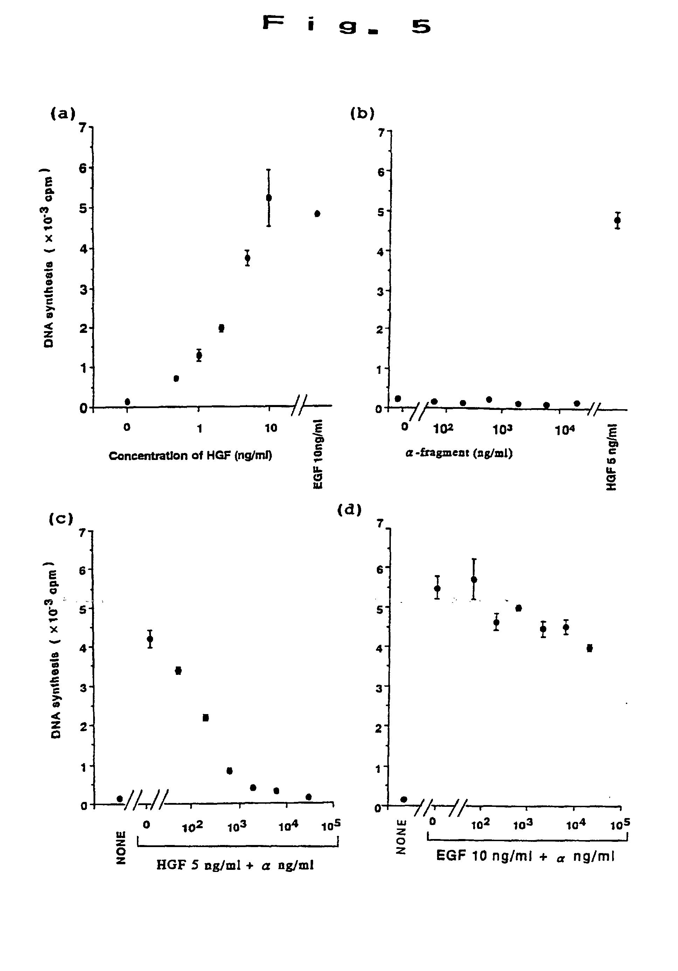

Rat liver cells were cultivated on 38 well plates so as to occupy an area of about 50%, and HGF, EGF and α-fragment were added, and cultivated for 22 hours. By labeling with ¹²⁵I-BrdU (0.15 mCi/well) for 4 hours, the activity was measured by scintillation counter. Results are shown in Fig. 5. In a range of 10² ng/ml to 10⁴ ng/ml of α-fragment, the DNA synthesis promoting action was not recognized. When added simultaneously with 5 ng/ml of HGF, it was known that the mitogen activity of HGF was suppressed dose-dependently (see Fig. 5c). Hence, α-fragment was proved to be an antagonist to mitogen and motogen activity of HGF.

### Example 4

### Action of α-fragment on invasion of cancer cells induced by HGF

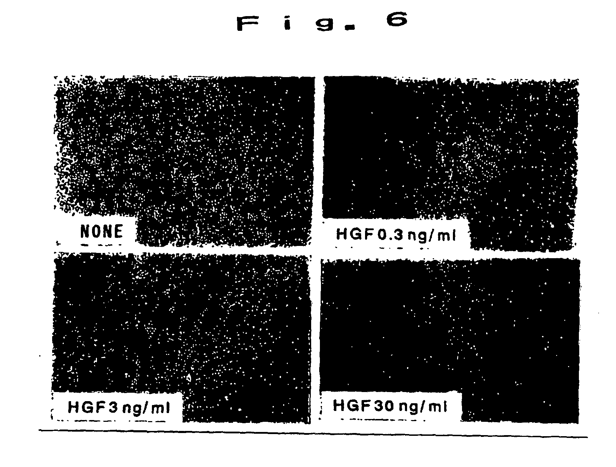

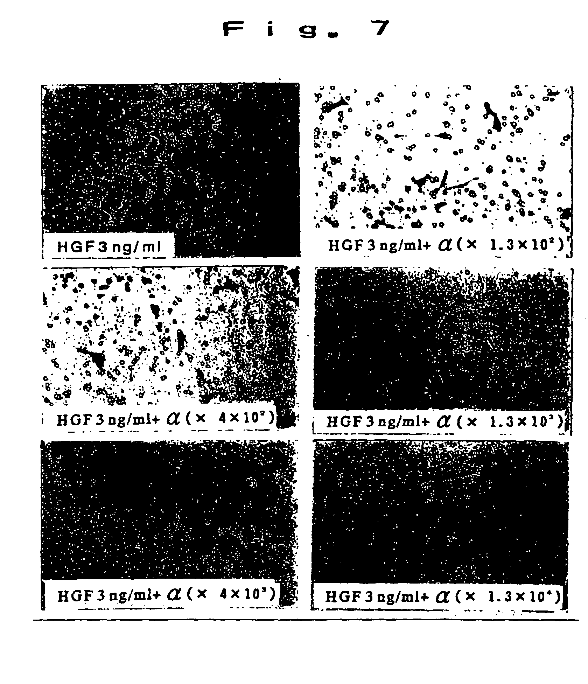

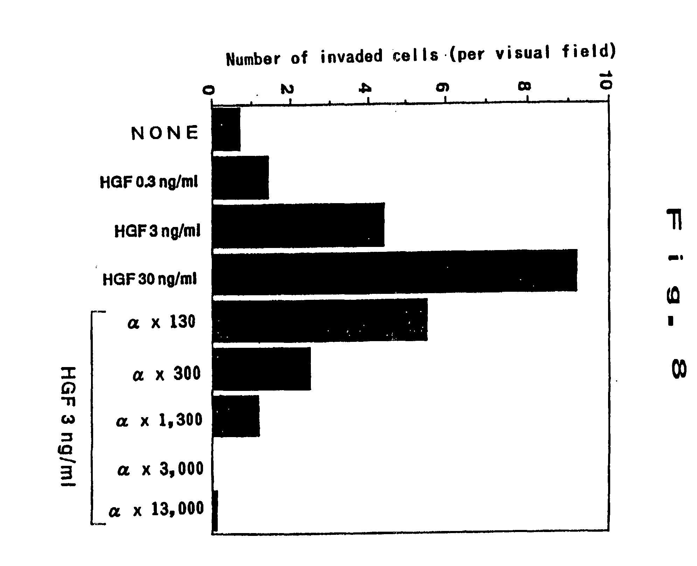

In the basal film invasion model using quantitative Matrigen invasion chamber (24 wells), the effect of α-fragment on the cancer cell invasion inducing action of HGF was studied. Adjusting GB-d1 cells to 1.5 x 10⁴ cells/200 ml/well, cells were seeded in the upper chamber. In 500 ml of culture medium of lower chamber, 2 ng/ml of HGF and α-fragment were added, and cultivated for 24 hours, and stained by H&E after fixing. Microscopically, ten arbitrary fields (8.4 mm²) were observed, and the ability of invasion was evaluated by the number of cells invading into the lower surface of the filter. As a result, HGF promoted invasion of GB-d1 cells dose-dependently (see Fig. 6). The action of α-fragment on 3 ng/ml of HGF was studied, and α-fragment inhibited the action of HGF dose-dependently, that is, 130 times, 400 times, 4000 times, 13000 times of 3 ng/ml of HGF (see Fig. 7). Results of Fig. 6 and Fig. 7 are graphically expressed in Fig. 8.

It is known from these results that α-fragment is effective for suppressing invasion of cancer cells induced by HGF.

### Preparation example 1

A solution containing 1 mg of α-fragment, 1 g of mannitol and 10 mg of polysorbate 80 in 100 ml of physiological saline was aseptically prepared, and poured into 1 ml vials, and freeze-dried and sealed, and freeze-dry preparations were obtained.

### Preparation example 2

An aqueous solution containing 1 mg of α-fragment and 100 mg of human serum albumin in 100 ml of 0.02M phosphate buffer solution (containing 0.15M NaCl and 0. 01% polysorbate 80, pH 7.4) was aseptically prepared, and poured into 1 ml vials, and freeze-dried and sealed, and freeze-dry preparations were obtained.

### Preparation example 3

A solution containing 1 mg of α-fragment, 2 g of sorbitol, 2 g of glycine and 10 mg of polysorbate 80 in 100 ml of distilled water for injection was aseptically prepared, and poured into 1 ml vials, and freeze-dried and sealed, and freeze-dry preparations were obtained.

## Claims

1. An anti-cancer agent containing a protein having the following physicochemical properties and physiological activities as an active ingredient:
a) composed of the α-chain of HGF obtainable by digesting HGF with elastase;
b) having a molecular weight of 55-69 kDa; and
c) having antagonist activity of c-Met/HGF receptor.

2. The anti-cancer agent of claim 1, wherein the HGF is manufactured by gene recombination method.

3. The anti-cancer agent of claim 2, wherein the host cell of gene recombination method is any one of Escherichia coli, Bacillus subtilis, yeast, filamentous fungi, and plant or animal cells.

4. Use of a protein as defined in claim 1 for manufacturing an anti-cancer agent.

5. The use of protein of claim 4, wherein HGF is manufactured by gene recombination method.

## Patentansprüche

1. Krebsmedikament, das als Wirkstoff ein Protein mit den folgenden physikalisch-chemischen Eigenschaften und physiologischen Aktivitäten enthält:
a) es besteht aus der α-Kette von HGF, die durch Verdauen von HGF mit Elastase erhältlich ist;
b) es hat ein Molekulargewicht von 55-69 kDa; und
c) es hat eine c-Met/HGF-Rezeptor-antagonistische Aktivität.

2. Krebsmedikament gemäß Anspruch 1, wobei HGF durch ein Genrekombinationsverfahren hergestellt ist.

3. Krebsmedikament gemäß Anspruch 2, wobei die Wirtszelle des Genrekombinationsverfahrens ausgewählt ist aus *Escherichia coli, Bacillus subtilis,* Hefe, Fadenpilzen und Pflanzen- oder Tierzellen.

4. Verwendung eines Proteins gemäß Anspruch 1 zur Herstellung eines Krebsmedikaments.

5. Verwendung eines Proteins gemäß Anspruch 4, wobei HGF durch ein Genrekombinationsverfahren hergestellt ist.

## Revendications

1. Agent anticancéreux contenant une protéine ayant les propriétés physicochimiques et les activités physiologiques suivantes en tant qu'ingrédient actif :
a) il est composé de la chaîne a de HGF que l'on peut obtenir en digérant le HGF avec une élastase ;
b) il a un poids moléculaire de 55-69 kDa ; et
c) il a une activité antagoniste du récepteur c-Met/HGF.

2. Agent anticancéreux selon la revendication 1, dans lequel le HGF est fabriqué par un procédé de recombinaison génétique.

3. Agent anticancéreux selon la revendication 2, dans lequel la cellule hôte du procédé de recombinaison génétique est l'une quelconque des cellules d'*Escherichia coli,* de *Bacillus subtilis,* de levure, de champignons filamenteux et des cellules végétales ou animales.

4. Utilisation d'une protéine telle que définie dans la revendication 1 pour la fabrication d'un agent anticancéreux.

5. Utilisation d'une protéine selon la revendication 4, dans laquelle le HGF est fabriqué par un procédé de recombinaison génétique.
